# EUROPÄISCHE PATENTSCHRIFT

(11) **EP 0 906 905 B1**
(45) Veröffentlichungstag und Bekanntmachung des Hinweises auf die Patenterteilung: **30.07.2003**
(21) Anmeldenummer: 98118571.3
(22) Anmeldetag: 01.10.1998
(51) Int. Cl.: C07D 233/32, C07D 233/40, C07D 239/10

(54) **Verfahren zur Herstellung von cyclischen Harnstoffderivaten**
Process for the preparation of cyclic ureas
Procédé de préparation d'urées cycliques

(30) Priorität: 02.10.1997 DE 19743760
(43) Veröffentlichungstag der Anmeldung: 07.04.1999
(73) Patentinhaber: BASF AKTIENGESELLSCHAFT, 67056 Ludwigshafen (DE)
(72) Erfinder: Kramer, Andreas, Dr., 67251 Freinsheim (DE); Siegel, Wolfgang, Dr., 67117 Limburgerhof (DE)
(74) Vertreter: Kinzebach, Werner, Dr.

(56) Entgegenhaltungen:
- EP-A- 0 141 755
- EP-A- 0 323 647
- FR-A- 1 316 792
- FR-A- 1 320 260

## Beschreibung

Die Erfindung betrifft ein Verfahren zur Herstellung von cyclischen Harnstoffderivaten.

Cyclische Harnstoffderivate, insbesondere N,N'-Dimethylpropylenharnstoff (DMPU) und N,N'-Dimethylethylenharnstoff (DMEU) werden beispielsweise in Agro- und Pharmasynthesen als polar aprotische Speziallösungsmittel eingesetzt. Besonders in Reaktionen, in denen Carbanionen oder Carbanionenäquivalente involviert sind, kann durch DMEU oder DMPU das carcinogene Hexamethylphosphorsäuretriamid (HMPA) als Lösungsmittel ersetzt werden.

Außerdem weisen viele cyclische Harnstoffderivate pharmakologische Aktivität auf; sie beeinflussen insbesondere das Zentralnervensystem.

Darüber hinaus sind cyclische Harnstoffderivate auch in der Verfahrenstechnik, z. B. in der Gaswäsche einsetzbar.

Es sind verschiedene Verfahren zur Herstellung cyclischer Harnstoffderivate bekannt:

Bogatsky, A.V. et al. beschreiben in Synthesis (1982), S. 464 und 465, die Synthese von cyclischen N,N'-Dialkylharnstoffen durch Umsetzung von cyclischen Thioharnstoffen mit Alkylhalogeniden in Gegenwart wässriger NaOH-Lösung und katalytischer Mengen Benzyltriethylammoniumchlorid.

Aus Hussain, M.H. und Lien, E.J., J. Med. Chem. (1971), 14/2, S. 138 - 144, ist bekannt, cyclische Harnstoffe durch Umsetzung von N,N'-Dialkylalkylendiaminen mit Harnstoff herzustellen.

Dehmlow, E.V. und Rao, Y.R. beschreiben in Synthetic Communications (1988), 18/5, S. 487 - 494, die Herstellung von Dimethylethylenharnstoff (DMEU) und Dimethylpropylenharnstoff (DMPU) durch Alkylierung der entsprechenden cyclischen Harnstoffe unter Phasentransferkatalyse.

Aus Rajca, A. et al., Synthesis (1983), S. 1032 und 1033, ist bekannt, cyclische Dialkylharnstoffderivate durch Umsetzung von N,N'-Dialkylalkylendiaminen mit 2-Oxo-5-phenyl-1,3,4-oxathiazol oder 5-(2,4-Dichlorophenyl)-2-oxo-1,3,4-oxathiazol herzustellen.

Die EP-A-280 781 und die ihr zugrundeliegende deutsche Prioritätsanmeldung DE-A-37 03 389 beschreiben ein Verfahren zur Herstellung von N-Alkyl-N'-methyl-alkylenharnstoffen, insbesondere von N,N'-Dimethylalkylenharnstoffen durch Umsetzung der entsprechenden Alkylenharnstoffe mit Formaldehyd zu den N-Alkyl-N'-(hydroxymethyl)-alkylenharnstoffen und nachfolgende Reduktion dieser Verbindungen zu den entsprechenden N-Alkyl-N'-methylalkylenharnstoffen mit Ameisensäure.

Aus der europäischen Patentanmeldung EP-A-198 345 ist ein Verfahren zur Herstellung von 1,3-Dialkyl-2-imidazolidinonen bekannt. Hierbei werden N,N'-Dialkylethylendiamine in Gegenwart eines polaren Lösungsmittels bei einer Temperatur von >180 °C mit Harnstoff umgesetzt.

Die EP-A-249 136 beschreibt ein Verfahren zur Herstellung cyclischer Harnstoffe durch Umsetzung eines N,N'-Dialkylalkylendiamins mit Harnstoff in einem polaren Lösungsmittel bei einer Temperatur von >180 °C.

Aus der DE-A-25 55 582 ist ein Verfahren zur Herstellung von nichtcyclischen oder cyclischen Harnstoffen durch Pyrolyse von jeweils 2 Mol eines entsprechenden Carbamats bekannt.

Aus der DE-A-26 54 928 ist ein Verfahren zur Herstellung von N-methylierten Harnstoffen oder Alkylenharnstoffen bekannt, wobei, ausgehend von Harnstoffen oder Alkylenharnstoffen, die Stickstoffatome mit Formaldehyd im sauren Milieu in Gegenwart eines Hydrierkatalysators in einem Schritt alkyliert werden.

Die GB-B-1 517 820 beschreibt ein Verfahren zur Herstellung von N-Methyl-Harnstoffen durch Umsetzung eines Harnstoffs mit Formaldehyd in saurem Medium und Hydrierung der so erzeugten Methylolverbindung an metallischen Katalysatoren.

Aus der JP-A-60 204728 ist bekannt, Ethylenharnstoff mit Alkylierungsreagenzien, wie Dimethylsulfat, zu Dimethylethylenharnstoff umzusetzen.

Die EP-A-248 220 beschreibt ein Verfahren zur Herstellung cyclischer Harnstoffe durch direkte Umsetzung eines Diamins mit Phosgen in Gegenwart von Wasser und einem HCl-Akzeptor.

Die US-A-4 617 400 beschreibt ein Verfahren zur Herstellung von N,N'-Dimethylharnstoffderivaten durch Umsetzung einer cyclischen Harnstoffverbindung mit Formaldehyd in Gegenwart von Wasserstoff und einem Hydrierkatalysator. Die Reaktion wird in Gegenwart einer festen Säure durchgeführt.

Die US-A-2 422 400 beschreibt ein Verfahren zur katalytischen Hydrierung von 1,3-Dimethoxymethylimidazolidin-2-on, 1,3-Dihydroxymethylimidazolidin-2-on oder anderen N,N'-Dihydroxymethyl-substituierten cyclischen Harnstoffen. Die Umsetzung erfolgt bei 100 bis 125 °C in einem organischen Lösungsmittel, beispielsweise Methanol, bei einem Druck von etwa 140 bar. Als Katalysator dient ein metallischer Hydrierkatalysator, insbesondere ein nickelhaltiger Katalysator.

Aus der DE-A-44 25 696 ist ein Verfahren zur Herstellung 1,3-disubstituierter Imidazolidinone durch Umsetzung von Ethylencarbonat mit mindestens einer Verbindung der Formel RNH₂ bekannt, in der R für H, Alkyl, Aryl, Heteroalkyl, oder Heteroaryl stehen kann. Die Reaktion wird bei einer Temperatur von 150 bis 300 °C und einem Druck von etwa 50 bis 150 bar durchgeführt. Lösungsmittel oder Katalysatoren werden nicht eingesetzt. In einem Beispiel wird eine Ausbeute von 85 % angegeben, in den anderen 4 Beispielen liegen die Ausbeuten zwischen 42 und 75 %.

Aus der EP-B-356 973 und der ihr zugrundeliegenden deutschen Prioritätsschrift DE-A-38 29 848 ist ein Verfahren zur Herstellung von cyclischen N,N'-Dimethylharnstoffen durch Umsetzung cyclischer Alkylenharnstoffe mit Formaldehyd und überschüssiger Ameisensäure bekannt, wobei man anschließend die noch im Reaktionsgemisch enthaltene Ameisensäure entfernt, indem man sie mittels eines Katalysatorsystems aus einem tertiären Amin und einem Kupfersalz einer thermischen Zersetzungsreaktion unterwirft.

Die DE-A-37 44 120 beschreibt ein Verfahren zur Herstellung von 1,3-Dialkyl-2-imidazolidinonen durch Umsetzung von N,N'-Dialkylalkylendiaminen mit Kohlendioxid in der Gasphase in Gegenwart oxidischer Katalysatoren.

Vail, S.L. et al. beschreiben in J. Org. Chem. (1965), 30, S. 2179 - 2182, die Bildung von Kondensationsprodukten einer Reaktionsmischung aus Dimethylharnstoff und wässriger Glyoxal-Lösung bei Raumtemperatur.

Die oben aufgeführten bekannten Verfahren sind entweder für eine Durchführung im technischen Maßstab nicht relevant, in der Reaktionsführung aufwendig, wegen hohen Salzanfalls wirtschaftlich unattraktiv oder gehen von Einsatzstoffen aus, die wirtschaftlich nur schlecht zugänglich sind.

Aus der DE-A-38 00 083 ist ein Verfahren zur Herstellung von cyclischen N,N'-Dimethylharnstoffen durch katalytische Hydrierung von cyclischen Harnstoffen, die in α-Stellung zu den beiden Stickstoffatomen Hydroxygruppen tragen, bekannt. Als Hydrierkatalysator wird Palladium auf einem anorganischen Trägermaterial verwendet. Als geeignete Lösungsmittel werden Wasser und polare, organische Lösungsmittel, zum Beispiel Alkohole, genannt. Die aufgeführten Beispiele beziehen sich jedoch ausschließlich auf die Herstellung von N,N'-Dimethylpropylenharnstoff aus N,N'-Dimethylolpropylenharnstoff in wässriger Lösung. Die Hydrierung der Methylolverbindung erfolgt in Gegenwart von Phosphorsäure. Der Nachteil dieses Verfahrens besteht darin, dass das Reaktionsgemisch zur Entfernung der restlichen Phosphorsäure aufgearbeitet werden muss. Zudem sind Verbindungen, bei denen die Hydroxygruppen an Kohlenstoffatome des Ringes gebunden sind, auf diese Weise nur schlecht hydrierbar.

Die FR-A-1 316 792 beschreibt ein Verfahren zur Herstellung von 4,5-Dialkoxy-1,3-dialkyl-imidazolidin-2-onen durch Umsetzung eines 1,3-Dialkylharnstoffs mit Glyoxal in Gegenwart eines niederen Monoalkanols und einer Säure.

Die FR-A-1 320 260 beschreibt ein Verfahren zur Herstellung von 4,5-Dihydroxy- bzw. 4,5-Dialkoxyimidazolidin-2-onen durch Umsetzung eines substituierten Harnstoffs mit Glyoxal.

Die EP-A-0 141 755 beschreibt ein Verfahren zur Herstellung von 4,5-Dihydroxyimidazolidin-2-onen durch Umsetzung von Glyoxal mit gegebenenfalls substituiertem Harnstoff in Gegenwart von Orthophosphorsäure als Katalysator.

Die EP-A-0 323 647 beschreibt ein Verfahren zur Herstellung von cyclischen N,N-Dimethylharnstoffen durch katalytische Hydrierung von cyclischen Harnstoffen, die in α-Stellung zu den beiden Stickstoffatomen Hydroxygruppen tragen.

Aufgabe der vorliegenden Erfindung ist daher, ein verbessertes Verfahren zur Herstellung von cyclischen Harnstoffderivaten bereitzustellen. Insbesondere sollte es im Hinblick auf Einsatzstoffe, Reaktionsführung und/oder die erzielten Ausbeuten gegenüber dem Stand der Technik verbessert sein.

Diese Aufgabe wird gelöst durch ein Verfahren zur Herstellung von cyclischen Harnstoffderivaten der Formel I 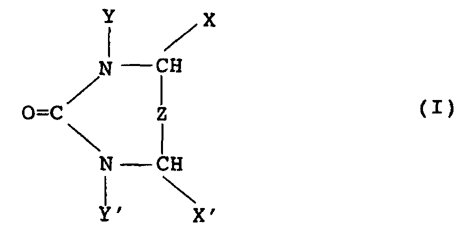 worin
- X und X': unabhängig voneinander für Wasserstoff, geradkettiges oder verzweigtes C₁-C₄-Alkyl, C₃-C₁₂-Cycloalkyl oder gegebenenfalls mit C₁-C₄-Alkyl substituiertes C₆-C₁₀-Aryl stehen;
- Y und Y': unabhängig voneinander für Wasserstoff, geradkettiges oder verzweigtes C₁-C₄-Alkyl, C₃-C₁₂-Cycloalkyl oder gegebenenfalls mit C₁-C₄-Alkyl substituiertes C₆-C₁₀-Aryl stehen, und
- Z: für eine Einfachbindung oder für einen gegebenenfalls mit einem Rest X oder X' substituierten C₁-C₄-Alkylenrest steht,
dadurch gekennzeichnet, dass man
a) ein Harnstoffderivat der Formel II 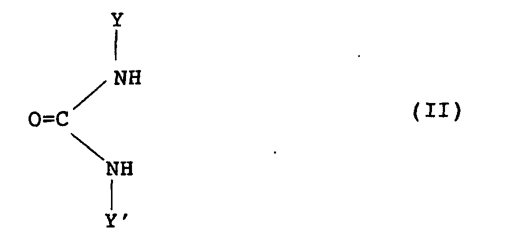 worin Y und Y' die oben angegebenen Bedeutungen besitzen, mit einem Diketon der Formel III 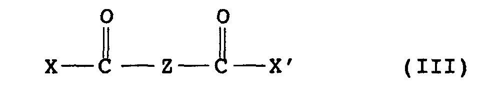 worin X, X' und Z die oben angegebenen Bedeutungen besitzen, umsetzt, und
b) das Reaktionsprodukt aus Schritt a) bei einer Temperatur von 130 bis 300 °C und bei einem Druck von 130 bis 320 bar in Gegenwart eines metallhaltigen Katalysators hydriert, wobei der Hydrierkatalysator ausgewählt ist unter Oxiden der ersten bis vierten Hauptgruppe des Periodensystems der Elemente, der ersten bis achten Nebengruppe des Periodensystems der Elemente und der Lanthanidengruppe oder deren Gemische sowie nicht geträgerten metallischen Kontakten oder metallischen Kontakten auf Trägern, ausgewählt unter Nickel, Kupfer und Cobalt.

Vorzugsweise sind die Reste Y und Y' unabhängig voneinander ausgewählt unter geradkettigem oder verzweigtem C₁-C₄-Alkyl oder C₆-C₁₀-Aryl, wie Methyl, Ethyl, n-Propyl, Isopropyl, n-Butyl, Isobutyl oder Phenyl, insbesondere unter Methyl oder Ethyl.

Die Reste X und X' sind vorzugsweise unabhängig voneinander ausgewählt unter Wasserstoff, geradkettigem oder verzweigtem C₁-C₄-Alkyl oder C₆-C₁₀-Aryl. Besonders bevorzugt sind X und X' Wasserstoff.

Z steht vorzugsweise für eine Einfachbindung oder für einen C₁-C₄-Alkylenrest, der mit einem oder mehreren der oben definierten Reste X oder X' substituiert sein kann, besonders bevorzugt für Methylen, Ethylen, Propylen oder Butylen oder insbesondere für eine Einfachbindung.

Das Diketon der Formel III kann auch in Form seiner chemischen Äquivalente, die unter den Reaktionsbedingungen das Diketon der Formel III freisetzen, eingesetzt werden.

Das erfindungsgemäße Verfahren weist gegenüber dem Stand der Technik folgende Vorteile auf:
- Verbindungen, die mit zu hydrierenden Gruppen substituiert sind, können mit hohen Ausbeuten hergestellt und hydriert werden.
- Die Herstellung solcher Verbindungen und deren Hydrierung kann kontinuierlich durchgeführt werden, wodurch die Wirtschaftlichkeit des Verfahrens erheblich gesteigert wird.

Vorzugsweise führt man den Reaktionsschritt a) in wässrigem oder wässrig-organischem Lösungsmittel durch. Es ist besonders bevorzugt, den Reaktionsschritt a) in wässrig-organischem Lösungsmittel durchzuführen, das etwa 1 bis 80 Gew.-%, insbesondere etwa 30 bis 60 Gew.-%, organisches Lösungsmittel, bezogen auf das Eduktgemisch, enthält. Das Reaktionsgemisch des Schrittes a) wird dann, gegebenenfalls nach weiterer Zugabe von organischem Lösungsmittel, hydriert.

Eine weitere Ausführungsform des erfindungsgemäßen Verfahrens ist dadurch gekennzeichnet, daß man nach Durchführung des Reaktions-Schrittes a) das Lösungsmittel entfernt, den Rückstand in organischem Lösungsmittel aufnimmt und hydriert.

Das Entfernen des wässrigen oder wässrig-organischen Lösungsmittels kann durch übliche Verfahren, beispielsweise durch Verdampfen des Lösungsmittels im Vakuum erfolgen.

Die aus Schritt a) resultierenden cyclischen Harnstoffderivate entsprechen insbesondere den Formeln IV, V oder VI 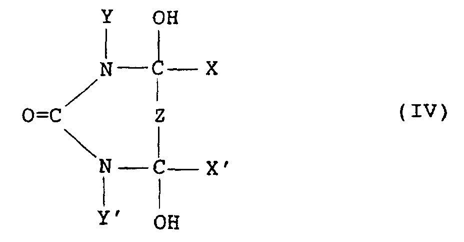 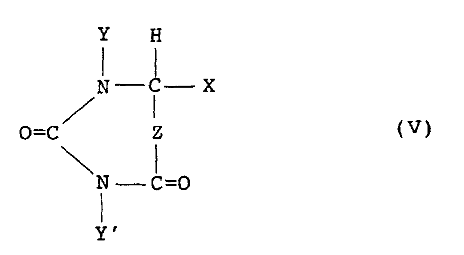 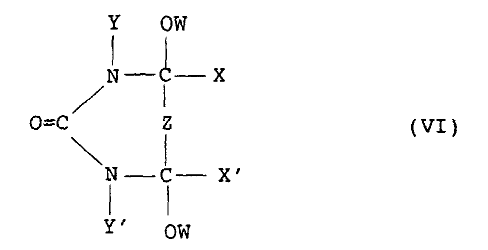 worin X, X', Y, Y' und Z die oben angegebenen Bedeutungen besitzen und W für einen geradkettigen oder verzweigten C₁-C₄-Alkylrest steht.

Als organisches Lösungsmittel verwendet man bevorzugt ein C₁-C₆-Alkanol, eine C₁-C₅-Carbonsäure, einen Ester einer C₁-C₃-Carbonsäure mit einem C₁-C₃-Alkanol oder einen linearen oder cyclischen Ether mit 3 bis 6 Gliedern. Erfindungsgemäß besonders bevorzugte organische Lösungsmittel sind Methanol, Ethanol, Propanol, Isopropanol, Butanol, Isobutanol, Essigester, Dioxan oder Eisessig, ganz besonders bevorzugt Methanol.

Der Reaktionsschritt a) kann im sauren, neutralen oder basischen Bereich ablaufen, vorzugsweise jedoch bei einem pH im Bereich von 3 bis 14, besonders bevorzugt 6 bis 10. Zur Einstellung des pH wird in Gegenwart geeigneter Mengen von Basen wie KOH, NaOH, Alkalicarbonaten, nicht-nucleophilen Aminen, Salzen aliphatischer Alkohole oder basischen Ionenaustauschern oder von Säuren, wie Ameisensäure, Schwefelsäure oder Phosphorsäure, organischen Sulfonsäuren oder von sauren Ionenaustauschern gearbeitet.

Der Reaktionsschritt a) kann in einem üblichen, druckfesten, beheizbaren Reaktionsgefäß, vorzugsweise im wässrig-alkoholischen, insbesondere wässrig-methanolischen Medium, durchgeführt werden. Reaktionstemperatur und Reaktionsdauer können in einem weiten Bereich variieren.

Die Reaktionstemperatur des Schrittes a) liegt im allgemeinen bei etwa 10 bis 100 °C, insbesondere etwa bei 20 bis 75 °C. Die Reaktionsdauer liegt im Bereich von einigen Minuten bis mehreren Stunden, insbesondere im Bereich von etwa 10 Minuten bis etwa 30 Stunden. Vorzugsweise werden die Verbindungen der Formeln II und III im molaren Verhältnis von etwa 1:1,5 bis etwa 1,5:1 eingesetzt.

Schritt b) wird erfindungsgemäß bei einer Temperatur von etwa 130 bis 300 °C, insbesondere etwa 150 bis 260 °C durchgeführt. Erfindungsgemäß arbeitet man in Schritt b) bei einem Druck von etwa 130 bis 320 bar, insbesondere etwa 150 bis 260 bar.

Das erfindungsgemäße Verfahren kann diskontinuierlich, halbkontinuierlich oder kontinuierlich durchgeführt werden, wobei die kontinuierliche Durchführung bevorzugt ist.

Erfindungsgemäß einsetzbar sind grundsätzlich alle Hydrierkatalysatoren. Als Hydrierkatalysatoren werden insbesondere Oxide der ersten bis vierten Hauptgruppe des Periodensystems der Elemente der ersten bis achten Nebengruppe des Periodensystems der Elemente und der Lanthanidengruppe oder deren Gemische, sowie nicht geträgerte metallische Kontakte oder metallische Kontakte auf Trägermaterialien eingesetzt. Bevorzugte geeignete Oxide der ersten bis vierten Hauptgruppe des Periodensystems der Elemente sind solche von Lithium, Natrium, Kalium, Calcium, Bor, Aluminium, Silicium und Zinn, vorzugsweise Zinn, Bor und Aluminium, insbesondere Zinn und Bor, der ersten bis achten Nebengruppe des Periodensystems der Elemente, wie Titan, Zirkon, Vanadium, Niob, Chrom, Molybdän, Mangan, Rhenium, Kobalt, Nickel, Ruthenium, Rhodium, Palladium, Osmium, Iridium, Platin, Kupfer und Silber, vorzugsweise Nickel, Kobalt, Ruthenium, Palladium, Kupfer oder Silber, besonders bevorzugt Nickel, Palladium, Kupfer und Silber und der Lanthanidengruppe, wie Lanthan, Praseodym, Samarium oder Ytterbium, vorzugsweise Praseodym oder deren Gemische.

Als metallische Kontakte auf Trägermaterialien eignen sich vorzugsweise Ruthenium, Nickel, Rhenium, Palladium, Platin, Kupfer, Cobalt, Rhodium, insbesondere Nickel, Palladium und Rhenium.

Die Katalysatoren können als Trägerkatalysatoren oder in kompakter Form, d. h. ohne Träger eingesetzt werden. Als Trägermaterialien können übliche Materialien verwendet werden, wie beispielsweise Bims, Siliciumdioxid, Aluminiumdioxid, wie α-, β- oder γ-Aluminiumoxid, Titandioxid, Zirkoniumdioxid, Magnesiumoxid, Silikate, Zeolithe oder Kohlenstoff in Form von Graphit, Aktivkohle oder Ruß, bevorzugt Aktivkohle, Aluminiumoxid, Siliciumdioxid und Titandioxid, besonders bevorzugt Aluminiumoxid, insbesondere α-Aluminiumoxid. Gegebenenfalls können zur Herstellung der Katalysatoren Bindemittel und Formhilfsmittel, wie Natriumcarbonat, Kaliumcarbonat, Natriumoxid und Magnesiumoxid, bevorzugt Natriumoxid mitverwendet werden. Das Aufbringen des Katalysators auf das Trägermaterial kann beispielsweise in üblicher Weise durch mehrfaches Tränken des Trägers mit einer wässrigen Lösung von Metallsalzen und zwischenzeitliches Trocknen sowie nachfolgende Überführung der Salze in die Oxide erfolgen. Geeignete Salze sind insbesondere Acetate, Carbonate oder Nitrate. Die Katalysatoren können auch als Festbett oder in Suspension eingesetzt werden. Gewünschtenfalls sind die Katalysatoren auch mit Seltenerdmetallen, wie Scandium, Yttrium oder Cer dotiert. Erfindungsgemäß wird der Katalysator in einer Menge von etwa 1 bis 30 Gew.-%, insbesondere etwa 10 bis 30 Gew.-%, bezogen auf das Reaktionsprodukt des Schrittes a), eingesetzt.

Die erfindungsgemäß hergestellten, cyclischen Harnstoffderivate sind als aprotische, polare Lösungsmittel zum Lösen von Arzneimitteln und von hochmolekularen Substanzen, insbesondere von Polyamiden, PVC, Polystyrol, Polyurethanen, Polyvinylalkohol oder Phenolharzen geeignet.

Die erfindungsgemäß hergestellten Harnstoffderivate sind außerdem als Wirkstoff in pharmazeutischen Mitteln, insbesondere in Mitteln, die das Zentralnervensystem (ZNS) beeinflussen, vorzugsweise in Antikonvulsiva, Antiepileptika, Antidepressiva, Sedativa, Relaxantien oder in Atmungs-Stimulantien verwendbar.

Ein weiterer Gegenstand der vorliegenden Erfindung ist ein cyclisches Harnstoffderivat der Formel I, das durch das erfindungsgemäße Verfahren erhältlich ist.

Die folgenden Beispiele erläutern die Erfindung, ohne sie jedoch darauf einzuschränken.

### Beispiele

### Beispiel 1:

### Kondensation von N,N'-Dimethylharnstoff mit Glyoxal

1a) Herstellung einer Verbindung der Formel IV, wobei X und X' für H stehen, Y und Y' für CH₃ stehen und Z für eine Einfachbindung steht.
   162,9 g N,N'-Dimethylharnstoff werden bei Raumtemperatur (RT) in 268,5 g 40%ige, wässrige Glyoxal-Lösung eingetragen. Nach 39 min Rühren wird in der Reaktionslösung ein pH-Wert von 6 bis 7 eingestellt und im Vakuum am Rotationsverdampfer bei 50 °C das Wasser entfernt. Der Rückstand wird in Ethanol aufgenommen und über Natriumsulfat getrocknet. Nach Austausch des Ethanols durch Aceton fällt beim Abkühlen auf 5 °C 1,3-Dimethyl-4,5-dihydroxy-2-imidazolidon (Komponente (1)) als weißer Feststoff aus (die Ausbeute liegt über 60 % d. Th.), der zum DMEU hydriert werden kann.
1b) Herstellung einer Verbindung der Formel V, wobei X für H steht, Y und Y' für CH₃ stehen und Z für eine Einfachbindung steht.
   162,9 g N,N'-Dimethylharnstoff werden bei RT in 268,5 g 40%ige, wässrige Glyoxal-Lösung eingetragen. Nach Zugabe von 188,5 ml Ameisensäure wird das Reaktionsgemisch 3 h zum Rückfluss gekocht. Anschließend wird die Ameisensäure unter 50 mbar Vakuum bei 50 °C entfernt und der Rückstand destillativ aufgearbeitet. Als Reaktionsprodukt wird mit einer Kopftemperatur von 135 bis 140 °C bei 20 mbar Vakuum 1,3-Dimethyl-imidazolidin-2,4-dion (Komponente (2)) (N,N'-Dimethylhydantoin) in einer Ausbeute über 70 % d. Th. gewonnen, das zum DMEU hydriert werden kann.
1c) Die Reaktanden werden wie unter 1a) zusammengegeben, jedoch erfolgt keine Isolierung einer einheitlichen Zwischenstufe. Das Reaktionsgemisch, das neben den Komponenten (1) und (2) noch weitere höhere Kondensationsprodukte enthält, wird nach Zugabe von Methanol direkt der Hydrierung zugeführt.
1d) Herstellung einer Verbindung der Formel IV, wobei X und X' für H stehen, Y und Y' für CH₃ stehen und Z für eine Einfachbindung steht.
   145 g einer 40 %-igen wässrigen Glyoxal-Lösung werden bei RT im Reaktionsgefäß vorgelegt. Mit Triethylamin wird ein pH von 8 eingestellt. Dann werden innerhalb von 100 min portionsweise 88 g Dimethylharnstoff als Feststoff zum Reaktionsgemisch gegeben. Nach einer Reaktionszeit von 3 h zwischen 20 und 40 °C wird im Reaktionsaustrag das 1,3-Dimethyl-4,5-dihydroxy-imidazolidon in einer Ausbeute von 95 % d. Th. gefunden. Der Reaktionsaustrag wird mit 230 g Methanol versetzt und kann ohne weitere Aufarbeitung in die Hydrierung eingesetzt werden.
1e) Herstellung einer Verbindung der Formel IV, wobei X und X' für H stehen, Y und Y' für CH₃ stehen und Z für eine Einfachbindung steht.
   1728 g einer 40 %-igen wässrigen Glyoxal-Lösung werden bei RT im Reaktionsgefäß vorgelegt, und mit 1 molarer methanolischer Natrium-Methylat-Lösung wird ein pH von 9 eingestellt. Zu diesem Gemisch wird eine Lösung aus 880 g Dimethylharnstoff in 880 g Methanol zugetropft. Nach einer Reaktionszeit von 1 h bei 40 °C wird im Reaktionsaustrag das 1,3-Dimethyl-4,5-dihydroxy-imidazolidon in einer Ausbeute von 83 % gefunden. Der Rohaustrag kann direkt in eine nachfolgende Hydrierung zum DMEU eingesetzt werden.

### Beispiel 2:

### Hydrierung der unter 1) beschriebenen Reaktionsprodukte zu DMEU (Herstellung einer Verbindung der Formel I, wobei X und X' für H stehen, Y und Y' für CH₃ stehen und Z für eine Einfachbindung steht.)

In einem 0,3 l Autoklaven werden 10 g Einsatzstoff (Reaktionsprodukte aus 1 a), b), c), d) und e)) in 100 ml Ethanol zusammen mit 1 bis 3 g Katalysator vorgelegt und in Anwesenheit von Wasserstoff bei einem Druck von 150 bis 200 bar und einer Temperatur von 160 bis 200 °C bis zur Druckkonstanz hydriert. Über die eingesetzten metallischen oder oxidischen Katalysatoren gibt Tabelle 1 Auskunft.

**Tabelle 1:**

| Ansatz Nr. | Katalysator | Edukt | Ausbeute |
|---|---|---|---|
| 1 | Raney-Nickel | aus 1a) | 90 % |
| | | aus 1b) | 87 % |
| | | aus 1d) | 80 % |
| | | aus 1e) | 80 % |
| 2 | 10,4 % Ni; 10,2 % Co; | aus 1a) | 83 % |
| | 4,2 % Cu auf Al₂O₃ | aus 1b) | 41 % |
| 3 | 50 % Ni; 30 % Zr; 18 % Cu; | aus 1a) | 48 % |
| | 1,5 % Mo; 0,2 % Na | aus 1b) | 86 % |
| | | aus 1c) | 73 % (in n-Propanol) |

### Beispiel 3: Kontinuierliche Hydrierung des Rohaustrags aus 1e)

Die hydrierende Umsetzung des Rohaustrags aus Beispiel 1e) wird in einem 0,3 l Rohrreaktor, in dem sich ein Katalysator in einem Festbett befindet, mit Kreisflüssigkeit durchgeführt. Der Reaktor wird über einen außenliegenden elektrischen Heizmantel auf die Reaktionstemperatur aufgeheizt. Die gasförmigen und flüssigen Einsatzstoffe durchströmen den Reaktor von unten nach oben (Sumpffahrweise). Der Hydrieraustrag wird entspannt und in einem Gas-Flüssigkeits-Abscheider in seine gasförmigen und flüssigen Bestandteile zerlegt. Ein Teil der im Abscheider gewonnenen Flüssigkeit wird in den Reaktor zurückgefahren, der andere in einen Austragsbehälter ausgetragen. Das Verhältnis von Zulauf zu Kreisflüssigkeit beträgt 1:10. Der Katalysator wird in Form von 2 bis 4 mm großen Strängen oder als Splitt eingesetzt und vor Beginn der Hydrierung mit Wasserstoff aktiviert. Der methanolische Zulauf enthält zwischen 11 und 23 % 1,3-Dimethyl-4,5-dihydroxy-imidazolidon.

In der Tabelle 2 werden die Reaktionsbedingungen sowie Selektivität und Umsatz wiedergegeben.

**Tabelle 2**

| Ansatz Nr. | Katalysator | Druck [bar] | Temperatur [°C] | Katalysatorbelastung (kg/l*h] | Umsatz [GC-Fl.-%] | Selektivität [GC-Fl.-%] |
|---|---|---|---|---|---|---|
| 1 | 10,4% Ni; 10,2% Co; 4,2% Cu auf Al₂O₃ | 166 | 175 | 0,018 | 90 | 80 |

## Patentansprüche

1. Verfahren zur Herstellung von cyclischen Harnstoffderivaten der Formel I 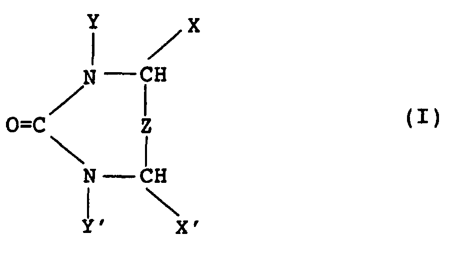 worin
X und X' unabhängig voneinander für Wasserstoff, geradkettiges oder verzweigtes C₁-C₄-Alkyl, C₃-C₁₂-Cycloalkyl oder gegebenenfalls mit C₁-C₄-Alkyl substituiertes C₆-C₁₀-Aryl stehen;
Y und Y' unabhängig voneinander für Wasserstoff, geradkettiges oder verzweigtes C₁-C₄-Alkyl, C₃-C₁₂-Cycloalkyl oder gegebenenfalls mit C₁-C₄-Alkyl substituiertes C₆-C₁₀-Aryl stehen, und
Z für eine Einfachbindung oder für einen gegebenenfalls mit einem Rest X oder X' substituierten C₁-C₄-Alkylenrest steht,
**dadurch gekennzeichnet, dass** man
a) ein Harnstoffderivat der Formel II 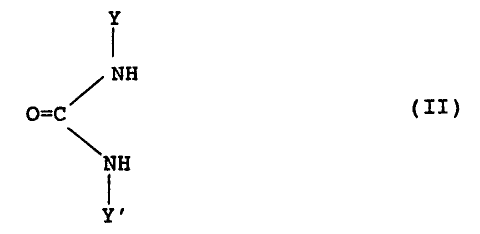 worin Y und Y' die oben angegebenen Bedeutungen besitzen, mit einem Diketon der Formel III 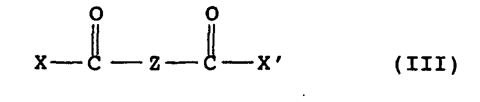 worin X, X' und Z die oben angegebenen Bedeutungen besitzen, umsetzt, und
b) das Reaktionsprodukt aus Schritt a) bei einer Temperatur von 130 bis 300°C und bei einem Druck von 130 bis 320 bar in Gegenwart eines metallhaltigen Katalysators hydriert, wobei der Hydrierkatalysator ausgewählt ist unter Oxiden der ersten bis vierten Hauptgruppe des Periodensystems der Elemente, der ersten bis achten Nebengruppe des Periodensystems der Elemente und der Lanthanidengruppe oder deren Gemische sowie nicht geträgerten metallischen Kontakten oder metallischen Kontakten auf Trägern, ausgewählt unter Nikkel, Kupfer und Cobalt.

2. Verfahren nach Anspruch 1, **dadurch gekennzeichnet, dass** man den Reaktionsschritt a) in wässrigem oder wässrig-organischem Lösungsmittel durchführt.

3. Verfahren nach Anspruch 1 oder 2, **dadurch gekennzeichnet, dass** man den Reaktionsschritt a) in einem wässrig-organischen Lösungsmittel durchführt, das etwa 1 bis 80 Gew.%, insbesondere etwa 30 bis 60 Gew.%, organisches Lösungsmittel, bezogen auf das Eduktgemisch, enthält.

4. Verfahren nach einem der vorhergehenden Ansprüche, **dadurch gekennzeichnet, dass** man nach Durchführung des Reaktionsschrittes a) das Lösungsmittel entfernt, den Rückstand in organischem Lösungsmittel aufnimmt und hydriert.

5. Verfahren nach Anspruch 2 oder 3, **dadurch gekennzeichnet, dass** man das Reaktionsgemisch des Schrittes a) gegebenenfalls nach weiterer Zugabe von organischem Lösungsmittel hydriert.

6. Verfahren nach einem der vorhergehenden Ansprüche, **dadurch gekennzeichnet, dass** man gemäss Schritt a) cyclische Harnstoffderivate der Formeln IV, V oder VI 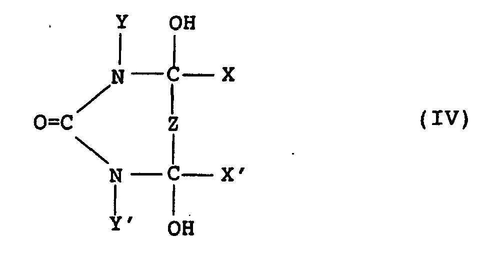 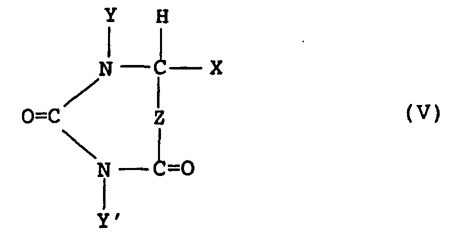 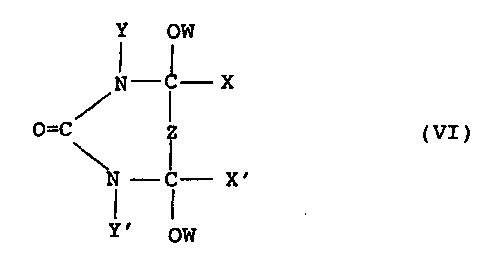 worin X, X', Y, Y' und Z die oben angegebenen Bedeutungen besitzen und W für einen geradkettigen oder verzweigten C₁-C₄-Alkylrest steht,
oder Gemische davon erhält.

7. Verfahren nach einem der vorhergehenden Ansprüche, **dadurch gekennzeichnet, dass** man als organisches Lösungsmittel ein C₁-C₆-Alkanol, eine C₁-C₅-Carbonsäure, einen Ester einer C₁-C₃-Carbonsäure mit einem C₁-C₃-Alkanol oder einen linearen oder cyclischen Ether mit 3 bis 6 Ringgliedern einsetzt.

8. Verfahren nach einem der vorhergehenden Ansprüche, **dadurch gekennzeichnet, dass** man Schritt a) bei einem pH im Bereich von 3 bis 14, insbesondere 6 bis 10 durchführt.

9. Verfahren nach einem der vorhergehenden Ansprüche, **dadurch gekennzeichnet, dass** man den Schritt b) bei einer Temperatur von 150 bis 260 °C durchführt.

10. Verfahren nach einem der vorhergehenden Ansprüche, **dadurch gekennzeichnet, dass** man den Schritt b) bei einem Druck von 150 bis 260 bar durchführt.

## Claims

1. A process for preparing cyclic urea derivatives of the formula I 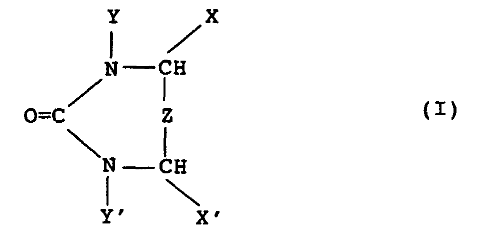 where
X and X' are, independently of one another, hydrogen, straight-chain or branched C₁-C₄-alkyl, C₃-C₁₂-cycloalkyl or unsubstituted or C₁-C₄-alkyl-substituted C₆-C₁₀-aryl;
Y and Y' are, independently of one another, hydrogen, straight-chain or branched C₁-C₄-alkyl, C₃-C₁₂-cycloalkyl, or unsubstituted or C₁-C₄-alkyl-substituted C₆-C₁₀-aryl, and
Z is a single bond or an unsubstituted or X- or X'-substituted C₁-C₄-alkylene radical,
which comprises
a) reacting a urea derivative of the formula II 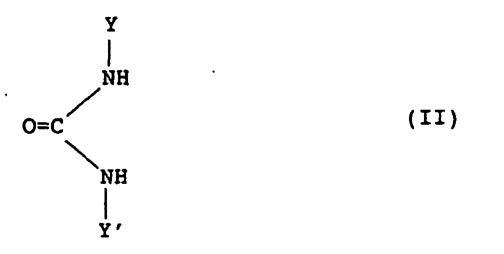 where Y and Y' have the abovementioned meanings, with a diketone of the formula III 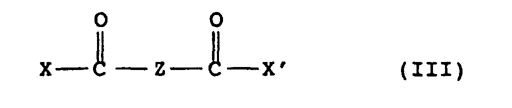 where X, X' and Z have the abovementioned meanings, and
b) hydrogenating the reaction product from step a) at a temperature of from 130 to 300°C under a pressure of from 130 to 320 bar in the presence of a metal-containing catalyst, where the hydrogenation catalyst is selected from oxides of group Ia to IVa of the periodic table of elements, of group Ib to VIIIb of the periodic table of elements and of the lanthanide group or mixtures thereof, and unsupported metallic catalysts or metallic catalysts on carriers, selected from nickel, copper and cobalt.

2. A process as claimed in claim 1, wherein step a) is carried out in aqueous or aqueous/organic solvent.

3. A process as claimed in claim 1 or 2, wherein step a) is carried out in an aqueous/organic solvent which contains about 1 - 80% by weight, in particular about 30 - 60% by weight, of organic solvent based on the precursor mixture.

4. A process as claimed in any of the preceding claims, wherein step a) is followed by removal of the solvent, and the residue being taken up in an organic solvent and hydrogenated.

5. A process as claimed in claim 2 or 3, wherein the reaction mixture from step a) is hydrogenated where appropriate after further addition of organic solvent.

6. A process as claimed in any of the preceding claims, wherein step a) results in cyclic urea derivatives of the formulae IV, V or VI 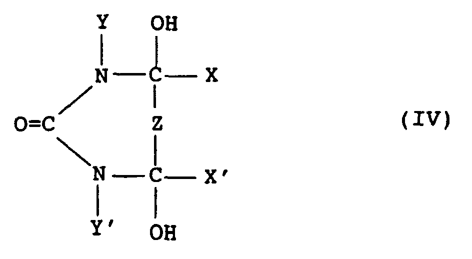 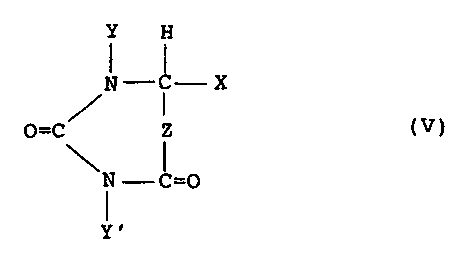 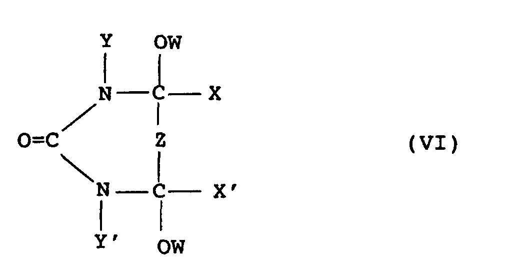 where X, X', Y, Y' and Z have the abovementioned meanigs, and W is a straight-chain or branched C₁-C₄-alkyl radical,
or mixtures thereof.

7. A process as claimed in any of the preceding claims, wherein a C₁-C₆-alkanol, a C₁-C₅-carboxylic acid, an ester of a C₁-C₃-carboxylic acid with a C₁-C₃-alkanol or a linear or cyclic ether with 3 to 6 ring members is employed as organic solvent.

8. A process as claimed in any of the preceding claims, wherein step a) is carried out at a pH in the range from 3 to 14, in particular 6 to 10.

9. A process as claimed in any of the preceding claims, wherein step b) is carried out at 150 - 260°C.

10. A process as claimed in any of the preceding claims, wherein step b) is carried out under a pressure of 150 - 260 bar.

## Revendications

1. Procédé de préparation de dérivés cycliques de l'urée de formule I 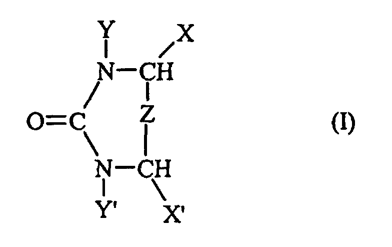 dans laquelle
X et X' représentent chacun indépendamment de l'autre un atome d'hydrogène ou un groupe alkyle en C₁-C₄ à chaîne droite ou ramifiée, cycloalkyle en C₃-C₁₂, ou aryle en C₆-C₁₀ éventuellement substitué par un ou des substituants alkyle en C₁-C₄ ;
Y et Y' représentent chacun indépendamment de l'autre un atome d'hydrogène, un groupe alkyle en C₁-C₄ à chaîne droite ou ramifiée, cycloalkyle en C₃-C₁₂ ou aryle en C₆-C₁₀ éventuellement substitué par un ou des groupes alkyle en C₁-C₄, et
Z est une liaison simple ou un radical alkylène en C₁-C₄ éventuellement substitué par un radical X ou X', **caractérisé en ce que**
a) on fait réagir un dérivé de l'urée de formule II 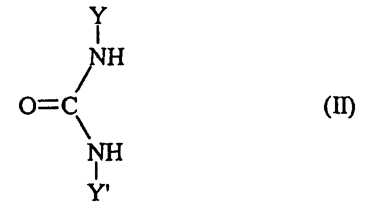 dans laquelle Y et Y' ont les significations données ci-dessus, avec une dicétone de formule III 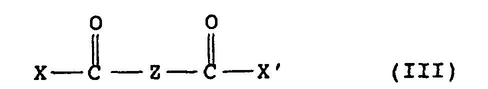 dans laquelle X, X' et Z ont les significations données ci-dessus, et
b) on hydrogène le produit de la réaction de l'étape a), à une température de 130 à 300°C et sous une pression de 130 à 320 bar, en présence d'un catalyseur contenant un métal, le catalyseur d'hydrogénation étant choisi parmi les oxydes du premier au quatrième groupes principaux du Tableau Périodique des Eléments, du premier au huitième groupes secondaires du Tableau Périodique des Eléments et du groupe des lanthanides, ou leurs mélanges, ainsi que les catalyseurs métalliques non supportés ou les catalyseurs métalliques supportés choisis parmi le nickel, le cuivre et le cobalt.

2. Procédé selon la revendication 1, **caractérisé en ce qu'**on met en oeuvre l'étape a) dans un solvant aqueux ou organique-aqueux.

3. Procédé selon la revendication 1 ou 2, **caractérisé en ce qu'**on met en oeuvre l'étape a) dans un solvant aqueux-organique, qui contient d'environ 1 à 80 % en poids, en particulier d'environ 30 à 60 % en poids de solvant organique par rapport au mélange de matières premières.

4. Procédé selon l'une des revendications précédentes, **caractérisé en ce que**, après mise en oeuvre de l'étape a), on élimine le solvant, on reprend le résidu dans un solvant organique, et on l'hydrogène.

5. Procédé selon la revendication 2 ou 3, **caractérisé en ce qu'**on hydrogène le mélange réactionnel de l'étape a), éventuellement après une nouvelle addition d'un solvant organique.

6. Procédé selon l'une des revendications précédentes, **caractérisé en ce qu'**on obtient selon l'étape a) des dérivés cycliques de l'urée de formules IV, V ou VI 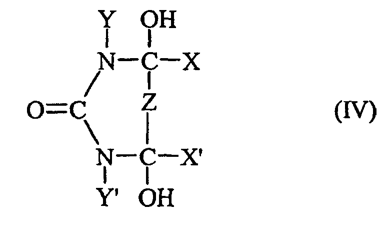 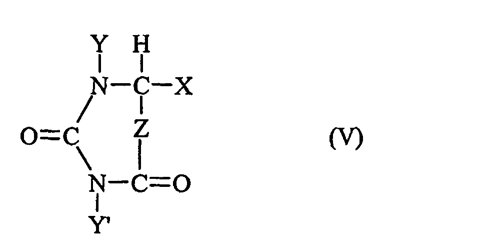 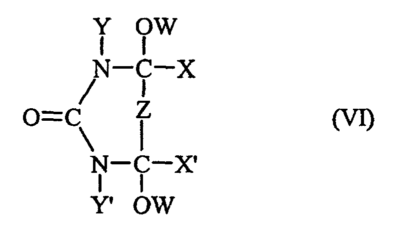 dans lesquelles X, X', Y, Y' et Z ont les significations données ci-dessus, et W est un radical alkyle en C₁-C₄ à chaîne droite ou ramifiée, ou des mélanges de ces dérivés.

7. Procédé selon l'une des revendications précédentes, **caractérisé en ce qu'**on utilise en tant que solvant organique un alcanol en C₁-C₆, un acide carboxylique en C₁-C₅, un ester d'un acide carboxylique en C₁-C₃ et d'un alcanol en C₁-C₃, ou un éther linéaire ou cyclique ayant 3 à 6 chaînons.

8. Procédé selon l'une des revendications précédentes, **caractérisé en ce qu'**on met en oeuvre l'étape a) à un pH compris entre 3 et 14, en particulier entre 6 et 10.

9. Procédé selon l'une des revendications précédentes, **caractérisé en ce qu'**on met en oeuvre l'étape b) à une température de 150 à 260°C.

10. Procédé selon l'une des revendications précédentes, **caractérisé en ce qu'**on met en oeuvre l'étape b) sous une pression de 150 à 260 bar.
